Europäisches Patentamt

European Patent Office

Office européen des brevets

(19)

(11) Publication number : **0 506 448 A1**

# (12) EUROPEAN PATENT APPLICATION

(21) Application number : **92302700.7**

(22) Date of filing : **27.03.92**

(51) Int. Cl.[5] : **C11D 3/386,** C12N 9/52, C12N 9/56

(30) Priority : **29.03.91 US 678866**

(43) Date of publication of application :
**30.09.92 Bulletin 92/40**

(84) Designated Contracting States :
**AT BE CH DE DK ES FR GB GR IT LI LU MC NL PT SE**

(71) Applicant : **The Clorox Company**
**1221 Broadway**
**Oakland California 94612 (US)**

(72) Inventor : **Anderson, Susan A.**
**3499 Edison Way**
**Menlo Park, California 94002 (US)**
Inventor : **Ottoboni, Susanne**
**2132 Lyon Avenue**
**Belmont, California 94002 (US)**
Inventor : **Tamm, Joseph A.**
**4830 Mulqueeney Common**
**Livermore, California 94550 (US)**

(74) Representative : **Froud, Clive et al**
**Elkington and Fife Prospect House 8**
**Pembroke Road**
**Sevenoaks, Kent TN13 1XR (GB)**

(54) **Alpha-lytic protease cleaning compositions.**

(57)   Cleaning compositions containing an alkaline serine protease from the genus Lysobacter are provided. Such cleaning compositions include variants of the alkaline serine protease which are characterized by a broad proteolytic activity, stability over broad pH and temperature ranges and stability to oxidizing agents.

EP 0 506 448 A1

The invention pertains to cleaning compositions, and to a method of cleaning using such compositions, which contain novel alkaline serine proteases originally derived from the genus Lysobacter.

A major trend in the detergent industry is to provide cleaning formulations that are functional at low wash temperature, are stable in the presence of laundry additives, and can be formulated as either liquid or granular compositions, while sufficient washing or cleaning ability is retained. These formulations generally require the use of enzymes, particularly proteases to fulfill this need.

In order to be useful as a detergent enzyme, it is desirable for a protease to possess proteolytic activity against proteinaceous substances over a wide pH and temperature range; exhibits catalytic stability in the presence of surfactants, builders, oxidizing agents and other detergent components; and maintains activity during storage (adequate shelf-life).

The most widely used proteases in cleaning compositions are the alkaline proteases derived from various strains of Bacillus. Such proteases, which are marketed by Novo/Nordisk Copenhagen, Denmark under trade names such as Esperase®, Alcalase®, or subtilisin Carlsberg, and Savinase®, derived from Bacillus licheniformis have desirable alkaline stability properties and proteolytic activities. These enzymes have been well characterized and are widely used by detergent manufacturers. While effective in hot water washing, the temperature optima of the Bacillus enzymes is between 60-70°C, which is above the normal temperatures used for warm (30-40°C) and cool (15-30°C) water washings. Moreover, the Bacillus alkaline proteases have less than desirable stability to oxidizing agents, and are completely unstable in chlorine bleaches, which precludes their use with chlorine bleaches, automatic dishwasher detergents, etc.

Variants of subtilisin, a serine protease produced by Gram-positive bacteria, have been produced using both random and site-directed mutagensis of the subtilisin gene to effect changes in catalytic efficiency (value of $K_{cat}/k_M$), substrate specificity and tertiary structure. Specific subtilisin variants have also been characterized which show improved stability to oxidation, increased protease activity or improved washing ability (U.S. Patent No. 4,760,025 issued 26 July 1988 to Estell, et al; WO89/06279 published 13 July 1989 to Hastrup, et al; and WO89/09819 published 19 October 1989 to Bryan, et al).

However, Estell, et al (1985) J Biol Chem 260:6518-6521 have shown that even for the relatively well characterized subtilisin protease (BPN'), choosing optimal amino acid substitutions for oxidatively sensitive residues (e.g., methionine at position 222) based on amino acid sequence homology to related proteases has not been predictive. Such oxidatively sensitive residues, even located at positions outside the active-site may be critical for catalytic activity due to as yet unknown structure-function relationships within the molecule.

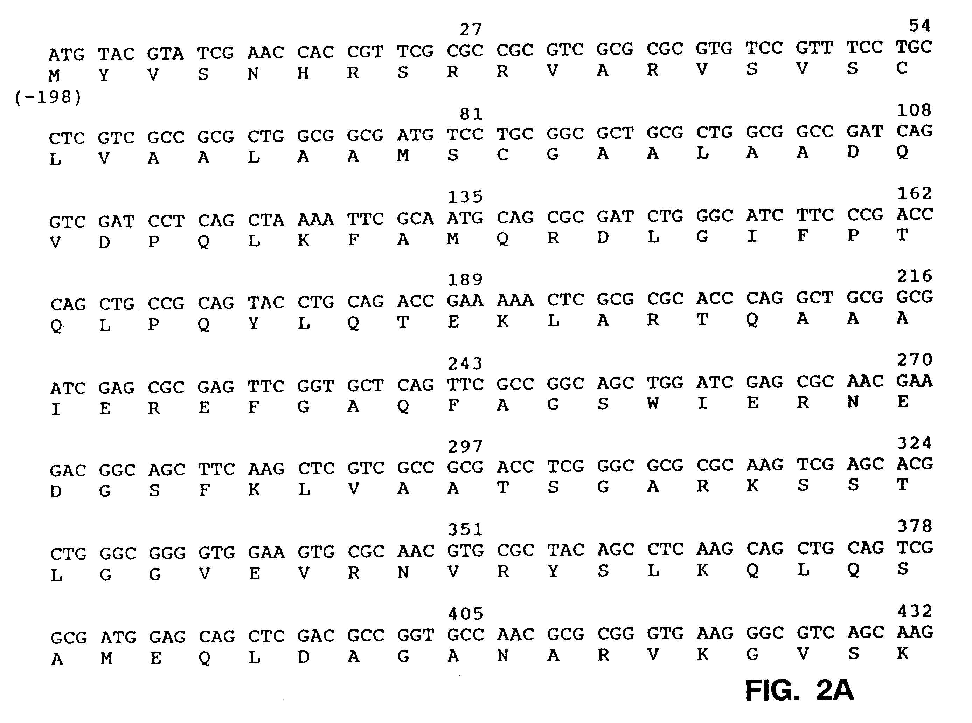

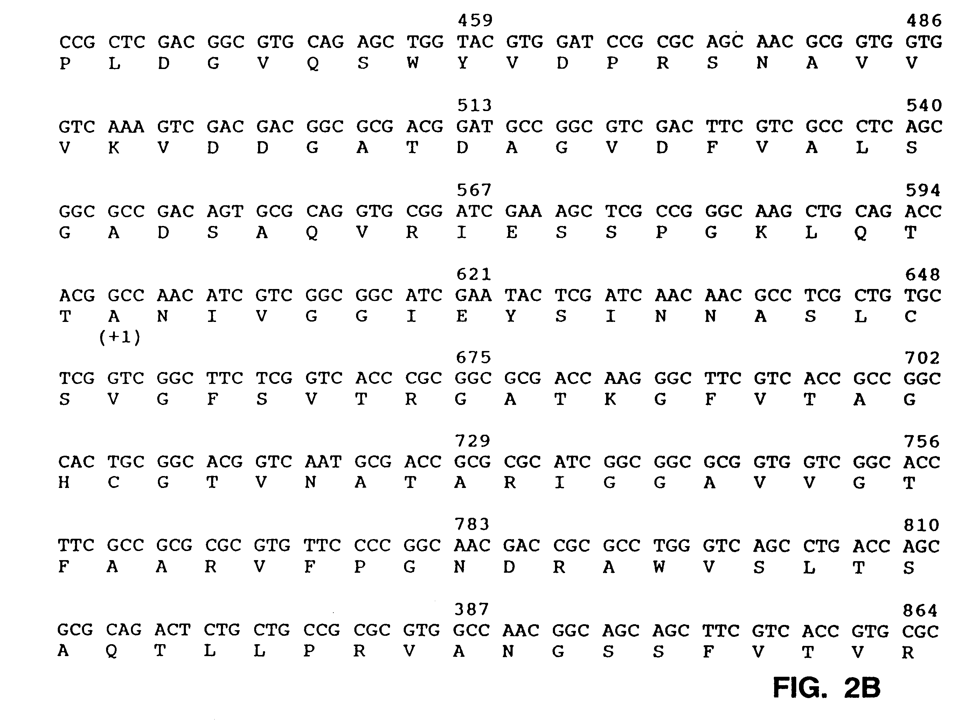

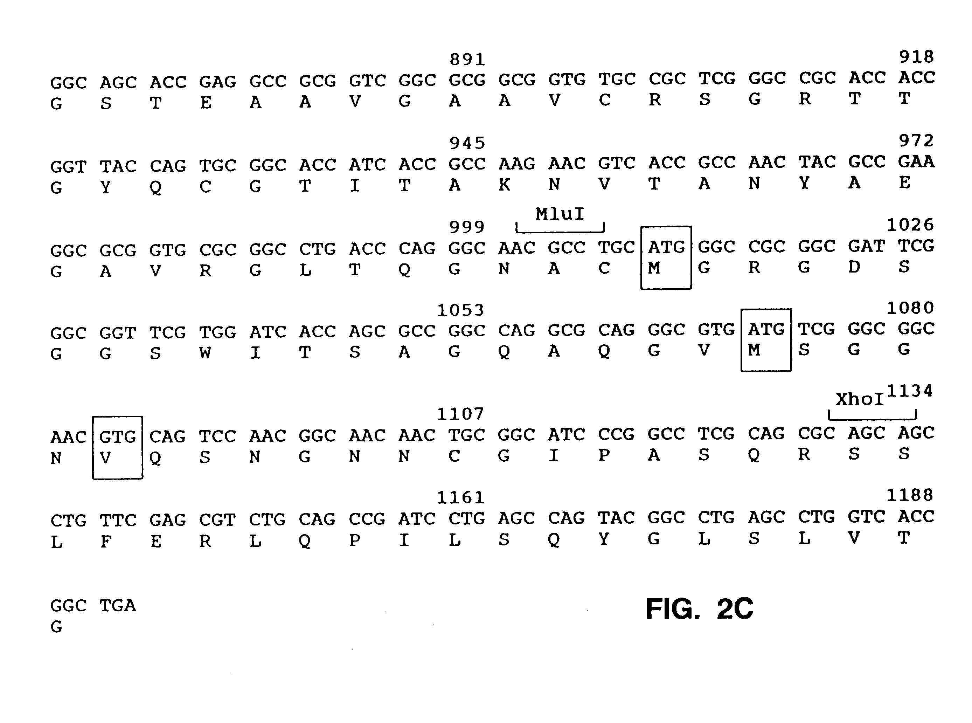

Previous work on alpha-lytic protease (ALP), a serine protease produced by Gram-negative bacteria such as, for example, Lysobacter enzymogenes, has led to the determination of the sequence of the gene (Epstein and Wensink (1988) J Biol Chem 263:16586-16590; Silen, et al (1988) Gene 69:237-244; also see FIG. 2 herein); the purification of the enzyme (Whitaker (1970) Methods in Enzymol 19:599-613); and the crystal structure of the protein (Brayer, et al (1979) J Mol Biol 131:743-775; Fujinaga, et al (1985) J Mol Biol 183:479-502).

From crystallographic data, a binding pocket important in directing substrate specificity includes methionine residues at positions 192 and 213 (numbered from 15-244 by alignment with chymotrypsin).

Variants of ALP, wherein alanine substituted for methionine at position 192 or 213, have been produced. Bone, et al ((1989) Nature 339:191-195) showed that the pattern of substrate specificity of alpha-lytic protease with respect to synthetic substrates, can be broadened by altering these methionine residues near the active-site. (For convenience, reference to natural amino acids may also be to the abbreviated one letter code.) For example, an alanine substitution for methionine at position 192 (MA192) results in broadened substrate specificity. The same substitution at position MA213 results in broader substrate specificity than the MA192 variant and also significantly lowers catalytic efficiency ($k_{cat}/K_m$). These results illustrate the complexity of enzyme-substrate interactions without illuminating any role for these modified enzymes in industrial applications.

The present invention resides in the discovery that certain alkalline serine proteases possess improved proteolytic activity toward proteinaceous stains, are stable over wide pH and temperature ranges and exhibit improved stability toward oxidizing agents. These characteristics make them well suited for formulation into laundry detergents, automatic dishwasher detergents, pre-soaks, laundry additives, as well as other types of cleaning compositions.

It has also been shown that the alpha-lytic proteases of the present invention are calcium-independent, that is, protease stability in solution in the absence of calcium is maintained. Unlike alkaline proteases of the subtilisin family, this characteristic makes the alpha-lytic proteases well suited for formulation into detergents containing builders (calcium sequesterants) used to control mineral hardness.

More specifically, the present invention provides a cleaning composition comprising a surfactant and in an amount effective to enhance removal of protein-containing materials, an alkaline protease having the sequence (numbered according to the native ALP sequence SEQ ID No: 2):

$$[AA_{1-137}]\text{-}Xaa_{138}\text{-}[AA_{139-157}]\text{-}Xaa_{158}\text{-}[AA_{159-162}]\text{-}Xaa_{163}\text{-}[AA_{164-198}]$$

wherein

$AA_{1-137}$ represent amino acids 1 through 137 of native alpha-lytic protease;

$Xaa_{138}$ is selected from the group consisting of methionine, serine and alanine;

$AA_{139-157}$ represent amino acids 139 through 157 of native alpha-lytic protease;

$Xaa_{158}$ is selected from the group consisting of methionine, alanine, threonine, glycine, serine and leucine;

$AA_{159-162}$ represent amino acids 159 through 162 of native alpha-lytic protease;

$Xaa_{163}$ is selected from the group consisting of valine and alanine; and

$AA_{164-198}$ represent amino acids 164-198 of native alpha-lytic protease.

Preferred embodiments of the alkaline protease described above are those wherein $Xaa_{138}$ is alanine; $Xaa_{158}$ is alanine; and $Xaa_{163}$ is alanine.

The alkaline protease used in formulating the cleaning compositions of the invention is originally derived from the genus Lysobacter. It has been discovered that variants of wild type alpha-lytic protease, wherein certain residues in the active-site have been replaced by generally conservative amino acid substitutions exhibit increased inherent thermal stabilities as well as improved soil removal performance against proteinaceous stains on fabric. In this embodiment of the invention is presented a cleaning composition wherein the alkaline serine protease has the following characteristics:

a) an optimum hydrolytic activity at a pH in the range of from about pH 7 to 12;

b) a stable activity in aqueous solution over a pH range of about pH 5 to 10.5; and

c) a melting temperature ($T_m$) greater than about 75°C at pH 5 and greater than about 73°C at pH 8.

In other embodiments the invention relates to a method of cleaning comprising contacting a surface soiled with proteinaceous material with a cleaning effective amount of the cleaning compositions provided above.

In yet another embodiment, the invention relates to a method of removing protein-soiled materials from a surface comprising contacting the surface with a cleaning composition of the invention containing in an amount effective to enhance removal of said protein-soiled materials.

In specific embodiments, the alkaline protease is originally derived from Lysobacter enzymogenes and may include amino acid substitutions at positions 138, 158, 163 or combinations thereof.

Referring to the accompagnying drawings:

FIG. 1 is a functional map of expression vector pALP5 which was used to express the various ALP variants. The MluI and XhoI restriction sites designated therein are convenient sites for inserting a DNA restriction fragment containing one or more modified codons of the ALP gene sequence.

FIG. 2 is a representation of the DNA sequence (top line) of the native, prepro-ALP and the corresponding amino acid sequence (bottom line). The mature protein begins at the designated (+1) alanine residue. The two overlining sections of nucleotides designates the MluI and XhoI restriction sites and the boxed areas represent the sites of amino acid substitutions.

FIG. 3 is a graph of the hydrolytic activity of wild-type ALP using a synthetic substrate, succinyl-Ala-Pro-Ala-para-nitroanilide, which was determined as a function of temperature.

As used in this invention the term "alpha-lytic protease" or "ALP" refers to a calcium-independent alkaline protease having a tertiary structure (e.g., a crystalline form) as set forth in Bone, et all (1987) Biochem 26:7609-7614. The primary sequence of the native, mature protein is provided in FIG. 2, beginning at residue +1. The term "alkaline" refers to a pH optimum of catalytic activity in the alkaline range, e.g., pH 7.0 to 11.0.

The primary sequence of ALP provided in FIG. 2 is numbered in accordance to the number of residues present in the mature protein. However, for purposes of the present invention, reference may be made to ALP variants having substitutions at residues "192", "213" and "217a". These numbers refer to alignment of the ALP sequence with the well characterized protease chymotrypsin. Thus, residue 138 of ALP aligns with 192 of chymotrypsin, while residue 158 of ALP aligns with 213, and residue 163 aligns with 217.

As used herein, an alkaline protease "derived from" a particular microorganism refers to the native alpha-lytic enzyme first identified by Whitaker (1970) Methods Enzymol 19:599-613. The derived enzyme is not necessarily physically derived from the microorganism but may be generated in any manner including, for example, expression from a recombinant microbial host.

Although there may be slight variations in a distinct type of naturally occurring enzyme within a given species of organism, enzymes of a specific type produced by organisms of the same species generally are substantially identical with respect to substrate specificity, thermal stability, activity levels under various conditions (e.g., temperature and pH), oxidative stability, and the like. Such characteristics of a naturally occurring enzyme such as ALP are not necessarily optimized for industrial applications. While advances in genetic engineering have provided methodology for altering various characteristics of protein catalysts, the understanding is limited to empirical determinations. That is, any specific amino acid substitution may result in subtle alterations in structure resulting in unexpected changes in kinetic parameters, physical properties and autolytic activity. It is therefore desirable to modify one or more native characteristics of ALP in order to deliver improvements over currently available commercial proteases for use in a specific cleaning environment.

While the native ALP enzyme has been discovered to be an improvement over commercially available proteases with respect to stability, the present invention also provides a number of ALP variants. These ALP variants (or their functional equivalents) useful in cleaning compositions exhibit the physical properties of the native enzyme considered to be advantageous for industrial applications. These include, without limitation, increased thermall stability, resistance to oxidation, and improved wash performance without sacrificing the catalytic properties of the native enzyme.

The ALP wild type and its variants exhibit enhanced thermal stability over native ALP or subtilisin. Thermal stability is a good indicator of the overall robustness of a protein in solution. Proteins exhibiting a high degree of thermal stability often are stable in the presence of chaotropic agents, detergents, and under other conditions which tend to inactivate proteins. Improvement of the inherent stability of proteins is therefore expected to be useful for many industrial applications in which resistance to high temperature, harsh solvent conditions or extended shelf-life is required. Stability has been addressed using mutagenesis, both site directed and through random methods, to enhance solution stability by modifying calcium binding to subtilisin proteases (see WO88/08033 issued to Amgen, Inc.). Using differential scanning calorimetry, protease stability can be evaluated by determination of the denaturation (melting) temperature $(T_m)$ of the enzyme. Further, it has been shown that for a series of over 300 subtilisin mutants, stability rankings obtained using differential scanning calorimetry to measure $T_m$ values correlated with long-term storage studies (see WO91/00334 to Casteleijn).

The DNA sequence encoding native ALP can be altered by nucleoide substitutions, or for the ALP variants by substitutions, additions or deletions that provide for functionally equivalent variant enzymes. For example, due to the degeneracy of the genetic code, other DNA sequences which encode substantially the same amino acid sequence as depicted in FIG. 2, including the target amino acid positions described herein, may be used to produce the proteases used in the cleaning compositions of the invention.

Thus, by using the information provided by identifying particular functions of certain residues of ALP, one can produce other variants having the properties similar to the native enzyme. For example, one or more amino acid residues at a non-critical region within the sequence can be substituted by another amino acid of a similar polarity which acts as a functional equivalent. Substitutes for an amino acid which should not affect the resulting activity of the enzyme are those where the residue is selected from other members of the class to which the target amino acid belongs, provided the substitution is not at an active or critical site of the enzyme.

In addition, one may combine substitutions to produce another class of variants having improved properties. For example, substitutions of more than one amino acid residue may be combined in a molecule using the information provided in Example 1 below. Or one may improve alpha-lytic proteases derived from other microorganisms which are closely related to Lysobacter. Closeness of relatedness or amino acid homology can be measured by comparison of the sequences. There are many methods of aligning protein sequences, but the differences are only manifest when the degree of relatedness is quite small. The methods described in Atlas of Protein Sequence and Structure, M.O. Dayhoff, editor, vol. 5, supplement 2 (1976) National Biomedical Research Foundation, Georgetown University Medical Center, Washington, D.C., p. 3 ff., entitled SEARCH and ALIGN, define relatedness.

As is well known in the art, related proteins can differ in the number of amino acids as well as the identity of each amino acid along the chain. That is, there can be deletions or insertions when two structures are aligned for maximum identity. Provided that the residue to be substituted in a target protease has a homologous counterpart to a residue in, for example, Lysobacter enzymogenes, the desirable mutations made in the ALP of Lysobactor enzymogenes can be made to the target protease to introduce one or more improved properties.

Development of ALP variants for purposes of the present invention is concentrated on targeting residues involved with the active-site region of ALP. The initial studies of Bone, et al (1989) supra (targeting the methionine residues at positions 192 and 213) addressed changes in substrate specificity using synthetic tetrapeptide derivatives. Whereas, the alteration in specificity against synthetic peptide substrates was shown, the utility of these modified proteases in practical settings against proteinaceous soils on fabric is not well understood. Specifically synthetic petide substrates allow quantification of a broadened specificity, but these measurable changes have not been shown to have any practical utility in predicting enzyme benefits (i.e., proteinaceous soil removal in a detergent matrix).

Techniques for the preparation of ALP analogs are well known to those skilled in the art and include site-directed mutagenesis (Sambrook, Fritsch & Maniatis, Molecular Cloning; A Laboratory Manual, Second Edition (1989)). This procedure involves modification, deletion and/or insertion of at least one amino acid at a predetermined site in the protease DNA sequence. For example, in a procedure known as cassette mutagenesis, restriction sites are introduced into the protease gene closely flanking the target codon or codons, and the fragment between the sites removed. Duplex synthetic DNA cassettes, designed to introduce an altered codon at the target site, are ligated into the restricted DNA to restore the coding sequence of the protease.

Another procedure employs the polymerase chain reaction (PCR) to amplify a portion of the desired gene (see U.S. Patent Nos. 4,683,202 and 4,965,188 which are incorporated herein be reference). By designing at leas: one primer which is not entirely complementary to the DNA strand to which it is to anneal, e.g., the mismatch incorporates the nucleotide(s) to be changed, one can readily amplify the desired DNA incorporating the codon change and subsequently ligate the amplified DNA product into a target gene sequence.

The proteases of this invention may be combined with detergents, builders, bleaching agents and other conventional ingredients to produce a variety of novel cleaning compositions useful in the laundry and other cleaning arts such as, for example, laundry detergents (both powdered and liquid), laundry pre-soaks, all fabric bleaches, automatic dishwashing detergents (both liquid and powdered), and household cleaners, particularly drain openers. In addition, the ALPs may also be employed in the cleaning of contact lenses and other membranes by contacting such materials with an aqueous solution of the cleaning composition.

The protease-containing cleaning compositions of the invention include laundry detergents, laundry pre-soaks, (bleaches) and automatic dishwashing detergents. The composition of such products is not critical to this invention, and the same may be readily prepared by combining an effective amount of the ALP enzyme preparation with the conventional components of such compositions in their art recognized amounts.

The cleaning compositions of the present invention include at least one surfactant selected from the anionic, cationic and nonionic surfactants. Particularly effective surfactants appear to be nonionic surfactants. Preferred surfactants of use include linear ethoxylated alcohols, such as those sold by Shell Chemical Company under the brand name Neodol. Other suitable nonionic surfactants can include other linear ethoxylated alcohols with an average length of 6 to 16 carbon atoms and averaging about 2 to 20 moles of ethylene oxide per mole of alcohol; linear and branched, primary and secondary ethoxylated, propoxylated alcohols with an average length of about 6 to 16 carbon atoms and averaging 0-10 moles of ethylene oxide and about 1 to 10 moles of propylene oxide per mole of alcohol; linear and branched alkylphenoxy (polyethoxy) alcohols, otherwise known as ethoxylated alkylphenols, with an average chain length of 8 to 16 carbon atoms and averaging 1.5 to 30 moles of ethylene oxide per mole of alcohol; and mixtures thereof.

Further suitable nonionic surfactants may include polyoxyethylene carboxylic acid esters, fatty acid glycerol esters, fatty acid and ethoxylated fatty acid alkanolamides, certain block copolymers of propylene oxide and ethylene oxide, and block polymers or pronylene oxide and ethylene oxide with propoxylated ethylene diamine. Also included are such semi-polar nonionic surfactants like amine oxides, phosphine oxides, sulfoxides, and their ethoxylated derivatives.

Anionic surfactants may also be suitable. Examples of such anionic surfactants may include the ammonium, substituted ammonium (e.g., mono-, di-, and triethanolammonium), alkali metal and alkaline earth metal salts of $C_6$-$C_{20}$ fatty acids and rosin acids, linear and branched alkyl benzene sulfonates, alkyl sulfates, alkyl ether sulfates, alkane sulfonates, olefin sulfonates, hydroxyalkane sulfonates, fatty acid monoglyceride sulfates, alkyl glyceryl ether sulfates, acyl sarcosinates and acyl N-methyltaurides.

Suitable cationic surfactants may include the quarternary ammonium compounds in which typically one of the groups linked to the nitrogen atom is a $C_{12}$-$C_{18}$ alkyl group and the other three groups are short chained alkyl groups which may bear inert substituents such as phenyl groups.

Further, suitable amphoteric and zwitterionic surfactants which contain an anionic water-solubilizing group, a cationic group and a hydrophobic organic group may include amino carboxylic acids and their salts, amino dicarboxylic acids and their salts, alkylbetaines, alkyl aminopropylbetaines, sulfobetaines, alkyl imidazolinium derivatives, certain quaternary ammonium compounds, certain quaternary phosphonium compounds and certain tertiary sulfonium compounds. Other examples of potentially suitable zwitterionic surfactants can be found described in U.S. Patent No. 4,005,029, at columns 11-15, which are incorporated herein by reference.

Further examples of anionic, nonionic, cationic and amphoteric surfactants which may be suitable for use in this invention are depicted in Kirk-Othmer, Encyclopedia of Chemical Technology, Third Edition, Volume 22, pages 347-387, and McCutcheon s Detergents and Emulsifiers, North American Edition, 1983, which are incorporated herein by reference.

Detergent compositions of the present invention can optionally comprise inorganic or organic detergent builders to assist in mineral hardness control. These builders can comprise from 0 to about 80% by weight of the composition. When included, these builders typically comprise up to about 60% by weight of the detergent composition. Built liquid formulations preferably comprise from about 10 to about 25% detergent builder while built granular formulations preferably comprise from about 10 to about 50% by weight detergent builder.

Suitable detergent builders include the crystalline aluminosilicate ion exchange materials disclosed in U.S. Patent No. 4,661,288 which is incorporated herein by reference.

Other examples of detergent builders include the various water-soluble, alkali metal, ammonium or substituted ammonium phosphates, polyphosphates, phosphonates, polyphosphonates, carbonates, silicates, borates, polyhydroxysulfonates, polyacetates, carboxylates, and polycarboxylates. Preferred are the alkali metal, especially sodium, salts of the above.

Other optional ingredients which can be included in detergent compositions of the present invention, in their conventional art-established levels for use (i.e., from 0 to about 20%), include solvents, bleaching agents, bleach activators, soil-suspending agents, corrosion inhibitors, dyes, fillers, germicides, pH adjusting agents (monoethanolamine, sodium carbonate, sodium hydroxide, etc.), enzyme stabilizing agents, perfumes, fabric softening components, static control agents, and the like.

Dyes include anthraquinone and similar blue dyes. Pigments, such as ultramarine blue (UMB), may also be used, and can have a bluing effect by depositing on fabrics washed with a detergent bleach containing UMB. Monastral colorants are also possible for inclusion.

Optical brighteners such as stilbene, styrene and styrlnaphthalene brighteners (fluorescent whitening agents), may be included. Fragrances used for esthetic purposes are commercially available from Norda, International Flavors and Fragrances and Givaudon. Stabilizers include hydrated salts, such as magnesium sulfate, and boric acid.

Granular or dry formulations embodying the detergent compositions of the present invention can be formed by conventional techniques, i.e., by slurrying the individual components in water and then atomizing and spray-drying the resultant mixture, or by pan or drum granulation of the ingredients. Granular formulations preferably comprise from about 10 to about 30% detergent surfactant, usually anionic.

Liquid formulations embodying the detergent compositions can be built or unbuilt. If unbuilt, these compositions conventionally contain approximately 15 to 50% total surfactant, from 0 to 10% of an organic base such as a mono-, di-, or tri-alkanol amine, a neutralization system such as an alkali metal hydroxide and a lower primary alcohol such as ethanol or isopropanol, and approximately 20 to 80% water. Such compositions are normaly homogeneous single phase liquids of low viscosity (approximately 100 to 150 centipoise at 75°F.). Preferably, liquid detergent compositions of the present invention contain from about 0.5-2.5% by weight of the protease enzyme preparation.

Built liquid detergent compositions can be in the form of single phase liquids provided that the builder is solubilized in the mixture at its level of use. Such liquids conventionallly contain 10 to 25% total surfactant, 10 to 25% builder which can be organic or inorganic, 3 to 10% of a hydrotrope system and 40 to 77% water. Liquids of this type also have a low viscosity (100 to 150 centipoise at 75°F). Built liquid detergents incorporating components that form heterogeneous mixtures (or levels of builder that cannot the completely dissolved) can also comprise detergent compositions of the present invention. Such liquids conventionally employ viscosity modifiers to produce systems having plastic shear characteristics to maintain stable dispersions and to prevent phase separation or solid settlement.

The following Examples illustrate the present invention:

Examples

Example 1. Site-Specific Mutagenesis, Expression, and purification of Alpha-Lytic Proteases

A. Mutagenesis and Expression of the Gene

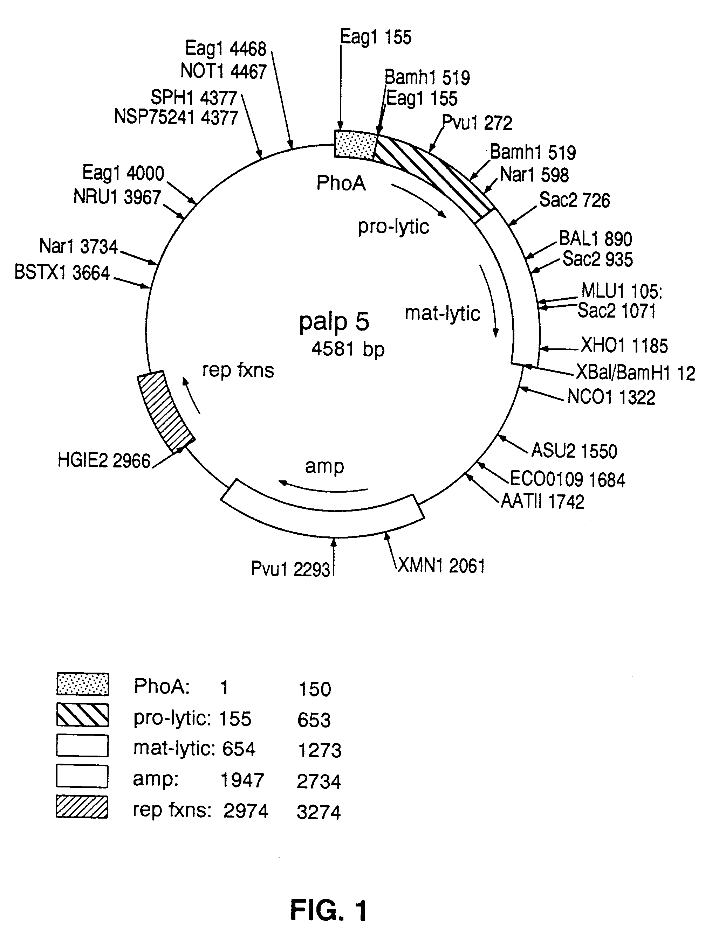

Variants of the ALP molecule that can hydrolyze a wide spectrum of peptide bonds with good efficiency were generated. Recent studies have demonstrated that two specific amino acids in the substrate binding pocket are important in determining the proteolytic specificity of ALP for synthetic substrates (Bone, et al (1989), supra). These residues are the methionine at position 192 (M192) and the methionine at position 213 (M213). In addition, the valine at position 217a (V217a) may play a role in defining the size of the substrate binding pocket. A collection of mutant ALP molecules containing specific amino acid changes at these three positions were constructed. The mutagenesis was carried out according to the manufacturer's instructions using the "Muta-Gene" mutagenesis kit from Bio-Rad. This kit is based on protocols that have been previously described by Kunkel, et al (1987) Methods in Enzymol 154:367-382). The plasmid pDA16XM (Silen, et al (1989) J Bacteriol 171:1320-1325) was employed as template for all annealing reactions. Folllowing identification of the desired mutations by DNA sequencing, each was subcloned into the expression vector pALP5 (Silen, et al (1985) supra; see FIG. 1) using the convenient MluI and XhoI restriction sites. The mutations made, as well as the oligonucleotides used to construct them, are listed below:

At position 192:

```
wild-type   5'- GCC GCG GCC CAT GCA CGC GT -3'
MG192       5'- GCC GCG GCC GCC GCA CGC GT -3'
MF192       5'- GCC GCG GCC GAA GCA CGC GT -3'
MD192       5'- GCC GCG GCC GTC GCA CGC GT -3'
MS192       5' -GCC GCG GCC CGA GCA CGC GT -3'
MA192       5' -GCC GCG GCC CGC GCA CGC GT -3'
```

At position 213:

```
wild-type   5'- GCC GCC CGA CAT CAC GCC CT -3'
MG213       5'- GCC GCC CGA GCC CAC GCC CT -3'
MS213       5'- GCC GCC CGA CGA CAC GCC CT -3'
ML213       5'- GCC GCC CGA CAG CAC GCC CT -3'
MT213       5'- GCC GCC CGA GGT CAC GCC CT -3'
MV213       5'- GCC GCC CGA CAC CAC GCC CT -3'
MF213       5'- GCC GCC CGA GAA CAC GCC CT -3'
MD213       5'- GCC GCC CGA GTC CAC GCC CT -3'
MA213       5'- GCC GCC CGA CGC CAC GCC CT -3'
```

At position 217a:

```
wild-type   5'- GTT GGA CTG CAC GTT GCC GC -3'
VS217a      5'- GTT GGA CTG CGA GTT GCC GC -3'
VA217a      5'- GTT GGA CTG CGC GTT GCC GC -3'
VF217a      5'- GTT GGA CTG GAA GTT GCC GC -3'
VD217a      5'- GTT GGA CTG GTC GTT GCC GC -3'
```

For each mutant listed in the above table, the first letter designates the amino acid present in the wild-type molecule; the second letter indicates the new residue that has been inserted and the number represents the position in the protein at which the change has been made. Thus, MG192 is a methionine to glycine change at position 192.

In addition to those mutations listed below, three double mutants were also constructed (MA192/MG213, MA192/VS217 MS213/VA217a). In two of these cases, the template for mutagenesis was not pDA16XM but rather a variant of this plasmid containing the MA192 mutation. This was obtained simply by subcloning a 762 base-pair BamHI fragment from pALP5MA192 into the BamHI site of M13mp18. The resulting phage DNA was then used as template for the MG213 and VS217 oligonucleotides to create the double mutants. These mutants were identified by DNA sequencing and subcloned into pALP5 as above. The third variant was identified by sequencing during screening for the MS213 mutant.

FIG. 2 is a representation of the DNA sequence of the native ALP gene, including the prepropeptide sequence, the coding sequence and the corresponding amino acid sequence. The prepropeptide sequence initiates at position (-198). Also designated in FIG. 2 are the variable codon and amino acid positions where substitutions have been made. Some of the substitutions made in the DNA were to introduce cleavage sequences recognized by certain restriction enzymes; these substitutions do not alter the amino acid corresponding thereto. Specifically, a C to G substitution at position 1005 was made to introduce a MluI restriction site, and an AGC to TCG substitution was made at nucleotides 1129-1131 to introduce an XhoI restriction site.

## B. Purification

This procedure describes a typical purification of a 10 liter scale fermentation of the wild-the ALP or the ALP variants, including the MA192 or MA213 enzymes.

Approximately 10 liters of fermentation broth were filtered with a 0.2 micron hollow fiber cartridge using an Amicon ultrafiltration system. The filtrate was then concentrated to approximately 1 liter using the same system and an Amicon S10Y3 cartridge (3,000 molecular weight cut-off "MWCO"). The ultrafiltrate was diafiltered against 15 liters of water using the same cartridge.

The ultrafiltrate concentrate was adjusted to pH 7.2 and adsorbed onto a 100 mL carboxymethylcellulose radial flow column. The protease was eluted from the column using 0.2 M sodium chloride in a 10 mM sodium phosphate buffer, adjusted to pH 7.2.

The fractions containing protease activity from the carboxymethylcellulose column were combined and diluted five-fold with deionized water, and the pH was adjusted to pH 5.5 with acetic acid. This material was applied to a 20 mL Mono S sulfopropyl cation-exchange collumn (pharmacia) equilibrated with 20 mM sodium acetate, adjusted to pH 5.5. The protease was eluted using a linear gradient of 0-0.25 M sodium chloride in 120 mL of 20 mM sodium acetate, pH 5.5

The protease-containing fractions from the Mono S cation-exchange collumn were combined and desalted using a mixed ion exchange resin (AG501-X8, Bio-Rad).

## Example 2. pH and Temperature Profiles

### A. Hydrolytic Activity Assay

The hydrolytic activity of each protease was measured using the preferred synthetic peptide para-nitroanilide substrate for the protease. For the wild-type alpha-lytic proteases, MG213, MT213, ML213, and MS213, the preferred substrate is succinyl-Ala-pro-Ala-para-nitroanilide (sAPApNA). For all other proteases examined, the preferred substrate is succinyl-Ala-Ala-Pro-Phe-para-nitroanilide (sAAPFpNA). The substrate concentration in the assay was 5 mM for sAPApNA, and 1 mM for sAAPFpNA.

The activity is assayed by monitoring the release of para-nitroaniline at 405 nm. The activity was determined spectrophotometrically in 1 or 3 mL cuvettes, using an extinction co-efficient = 10.4 mM$^{-1}$-cm$^{-1}$, or alternatively, with a microtiter platereader in a volume of 0.2 mL, using an extinction co-efficient = 5.84 mM$^{-1}$. The protease and substrate were diluted into 100 mM Tris buffer, adjusted to pH 8.0, to give a linear rate for at least 2 min. Assays were conducted at 25°C.

### B. Activity as a Function of PH

The optimum hydrolytic activity was determined by measuring the percent of the maximum activity (% of highest measured activity) of a given protease by varying the pH of the assay solution over the pH range 5.0 to 12.0. Optimum activity is defined as 30% or greater of maximal activity determined by the assay for hydrolytic activity described above.

The buffers used during each of these assays all contained 0.1 M KCl, and were as follows:

pH 3.8: 20 mM formate
pH 4.7: 20 mM acetate
pH 6.1: 20 mM MES
   (2-[N-morpholino]ethane sulfonic acid)
pH 6.8: 20 mM PIPES
   (1,4-piperazine diethane sulfonic acid)
pH 7.2: 20 mM phosphate
pH 8.0: 20 mM Tris
pH 8.4: 20 mM TAPS
   (Tris[hydroxymethyl]methylaminopropane sulfonic acid)
pH 9.2: 20 mM CHES
   (2-[N-cyclohexylamino]ethane sulfonic acid)
pH 9.8: 20 mM glycine
pH 10.4:20 mM carbonate
pH 12.0:20 mM phosphate

The results of these experiments are shown in Table 1.

## Table 1

### % of maximum activity

| Enzyme | pH | | | | | | | |
|---|---|---|---|---|---|---|---|---|
| | 5 | 6 | 7 | 8 | 9 | 10 | 11 | 12 |
| wt ALP | 2 | 19 | 75 | 88 | 95 | 99 | 100 | 95 |
| MA192 | 3 | 19 | 54 | 75 | 100 | 89 | 80 | 25 |
| MA213 | 2 | 21 | 71 | 84 | 89 | 91 | 100 | 79 |
| MS192 | 2 | 21 | 79 | 89 | 100 | 91 | 97 | 55 |
| MA192/MG213 | 4 | 20 | 62 | 82 | 100 | 94 | 100 | 62 |
| MS213/VA217a | 1 | 16 | 69 | 85 | 100 | 95 | 100 | 72 |
| MG213 | 1 | 8 | 45 | 59 | 92 | 99 | 100 | 92 |
| MT213 | 1 | 11 | 48 | 59 | 94 | 97 | 100 | 88 |
| ML213 | 2 | 16 | 63 | 65 | 98 | 96 | 100 | 88 |
| MS213 | 1 | 10 | 48 | 59 | 96 | 97 | 100 | 89 |
| Alcalase® | 2 | 14 | 50 | 72 | 95 | 100 | 82 | 9 |
| Savinase® | 4 | 4 | 5 | 52 | 94 | 100 | 96 | 2 |

The results show that at pH 6 to 8 alpha-lytic protease and all tested variant enzymes exhibit higher hydrolytic activity than Alcalase® or Savinase®. This is important for liquid laundry products where the wash water pH is much lower than for dry products. The wild-type ALP and the variants have optimal activities in the pH range of 7 to 12. All of the alpha-lytic proteases exhibit higher activity than Savinase® or Alcalase® at pH 12.

### C. Temperature Profile

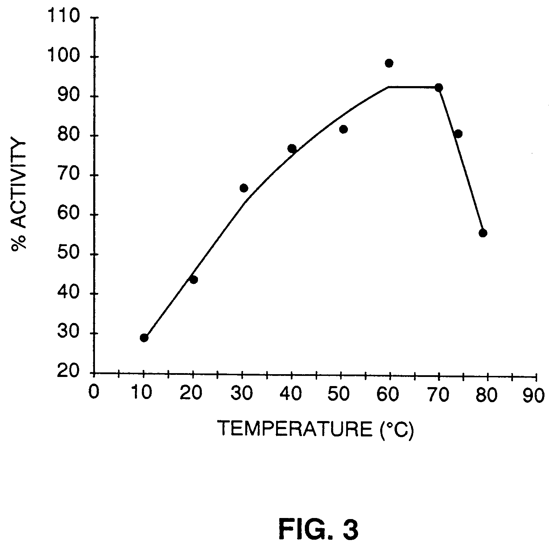

Using the hydrolytic activity assay, the activity of wild-type alpha-lytic protease was determined as a function of temperature by adding enzyme to buffered reaction solutions at pH 8 pre-equilibrated at the appropriate temperature. The results of this experiment are presented in FIG. 3.

The data demonstrates that alpha-lytic protease functions over a broad temperature range, with optimum activity (30% or greater) between 15 and 70°C.

### Example 3. Specific Proteolytic Activity Assay

The specific proteolytic activity of specific proteases was determined using casein as the substrate at pH 9. The assay measures trichloroacetic acid-soluble peptides released from casein during incubation with the protease under standard conditions (30 minute incubation at 25°C, pH 9.0) by reaction with the Folin-Ciocalteu reagent. Specific activity was determined by comparison with a tyrosine standard. One unit is defined as the amount of enzyme liberating peptides which give a response equivalent to 1 ug of tyrosine per minute. Specific activity was determined after incubation for 30 minutes at 25°C and pH 9.0. Results are presented in Table 2.

## Table 2

| Enzyme | Specific Activity ug Tyr/mg.min |
|---|---|
| wt alpha-lytic protease | 33 |
| MA192 | 13 |
| MA213 | 22 |
| Alcalase | 220 |
| Savinase | 270 |

Example 4. Thermal Stability

The stability of wild-the alpha-lytic protease and the mutants MA192 and MA213 in solution at 50°C was examined and compared to that of currently utilized detergent proteases Savinase® and Alcalase®. Enzymes were incubated at a concentration of 1 mg/mL at 50°C at three different pH values: pH 5 (50 mM acetate, 10 mM CaCl$_2$), and pH 8 (50 mM Bicine, 10 mM CaCl$_2$), and pH 10.5 (50 mM CAPS, 10 mM CaCl$_2$). Aliquots were removed at appropriate time points and assayed for activity against the preferred synthetic peptide substrate as desribed in the hydrolytic activity assay. Inactivation of all enzymes displayed first-order kinetics, and activity was monitored over at least two half-lives. The results are presented in Table 3.

## Table 3

| Enzyme | $t_{1/2}$ (hours) at 50°C | | |
|---|---|---|---|
| | pH 5 | pH 8 | pH 10.5 |
| wt ALP | 65 | 10 | 6 |
| MA192 | 170 | 28 | 6 |
| MA213 | 1000 | 110 | 8 |
| Savinase | 27 | 6 | 4 |
| Alcalase | 30 | 4 | 8 |

The data indicates that both mutants MA192 and MA213 are more stable than the wild-type enzyme at pH 5 and pH 8. In particular, MA213 is significantly more stable than the wild-the enzyme. Further, alpha-lytic proteases and the tested mutants are substantially more stable than either Savinase® or Alcalase® at pH 5 and pH 8. This is important for liquid cleaning compositions where the shelf-life of the product is limited due to enzyme instability. In contrast, the differences in stability among the proteases at pH 10.5 are less pronounced.

The thermal stability of the proteases was also examined by differential scanning calorimetry in order to determine the denaturation temperatures of the proteins. The denaturation temperature is directly related to the thermodynamic stability of a protein and provides a measure of resistance to unfolding. A comparison of denaturation temperatures ($T_m$) of similar proteins allows for ranking in order of thermal stability (Pantoliano, et al (1989) Biochem 28:7205-7213).

Calorimetric measurements were obtained using a Setaram Micro DSC at a scan rate of 0.7 degrees per minute. Proteases were inactivated prior to analysis to prevent autolysis using 1 mM phenylmethylsulfonyl fluoride for Alcalase® and Savinase®, and 1 mM diisopropylfluorophosphate for alpha-lytic protease and mutants. Calorimetric measurements were performed at a protein concentration of 2-4 mg/mL at pH 5.0 (50 mM acetate, 10 mM CaCl$_2$) or at pH 8.0 (50 mM Bicine, 10 mM CaCl$_2$). Denaturation temperatures were determined at the midpoint of the denaturation endotherm.

The results of these measurements are listed in Table 4.

Table 4

| Enzyme | $T_m$ (°C) | |
|---|---|---|
| | pH 5 | pH 8 |
| wt ALP | 78.6 | 73.2 |
| MA192 | 79.1 | 73.7 |
| MG213 | 80.0 | 73.0 |
| ML213 | 81.6 | nd |
| MT213 | 83.0 | nd |
| MA213 | 83.0 | 76.5 |
| Savinase | 73.4 | 71.4 |
| Alcalase | 71.5 | 69.1 |

nd = not determined

At both pH values, the higher denaturation temperatures of alpha-lytic protease compared to those of Savinase® and Alcalase® indicate that alpha-lytic protease is intrinsically more stable than either Savinase® or Alcalase®. The increased denaturation temperatures of the variants relative to the wild-type enzyme demonstrate that all these mutations produced increased thermal stability.

Example 5. Detergent Stability/Washability

The stability of the alpha-lytic proteases in typical dry and liquid detergents was compared to that of Alcalase® and Savinase®.

The enzymes were incubated at 100 ug/mL in the detergents at use levels for 12 minutes at 50°C (hot water wash). Residual activities at the end of incubation were determined by the casein activity assay. The results are presented in Table 5.

## Table 5

### % activity remaining after 12 min at 50°C

| Enzyme | Powdered[a] | Liquid[b] |
|---|---|---|
| wt ALP | 91 | 99 |
| MA192 | 75 | 78 |
| MA213 | 83 | 80 |
| Alcalase | 21 | 60 |
| Savinase | 33 | 94 |

[a] 9.1% surfactant, 77.9% builders and 13% other, including bleaching agents, fabric whitening agents and minor components

[b] 32.5% surfactant, 1.1% builders, 52.4% $H_2O$ and 14% other, including fabric whitening agents, solvents, stabilizers and minor components.

The results demonstrate that alpha-lytic protease and the tested mutants retain at least 75% of their initial activity under a typical hot water wash condition. Under alkaline wash conditions (i.e. a powdered detergent), the level of activity is at least twice that of Alcalase® or Savinase®. Enhanced stability is also observed compared to Alcalase in a liquid detergent.

The wash performance of alpha-lytic protease and mutants was compared to that of the commercially utilized detergent proteases Alcalase®, Savinase®, and Esperase® in beaker-scale studies (See Table 6). The protein levels used in these studies were determined by equall proteolytic activity against casein. The proteases were assayed for grass stain removal from cotton swatches at 25°C in a liquid detergent solution (Wisk®, Lever Bros., 19% anionic surfactant (NaLAS), 7% nonionic surfactant (AEO), 7% hydrotropes, 10% builder (Nacitrate), 0.09% brightners, 56% $H_2O$ and 0.1% others) at use level (1.9 g/L), adjusted to pH 9 or pH 10.5. Results are expressed as percent stain removal over a control containing no enzyme, and are summarized in Table 6.

## Table 6

| Enzyme | Δ % SRE | |
|---|---|---|
| | pH 9 | pH 10.5 |
| wt ALP | 5.3 | 0 |
| MA192 | 4.4 | 22.4 |
| MA213 | 9.7 | 23.6 |
| Alcalase | 11.2 | 11.8 |
| Esperase | 9.2 | 15.5 |
| Savinase | 6.8 | 14.9 |

The results of these experiments demonstrate that the alpha-lytic protease mutants MA192 and MA213 show an enhanced benefit at pH 10.5.

Grass stain removal was similarly compared on an equal weight basis (0.5 mg active protease/e) at 37°C in a solution prepared using a powdered detergent (104g/69 liters) at pH 10.5. These results are shown in in Table 7.

## Table 7

| Enzyme | Δ%SRE |
|---|---|
| Savinase | 51 |
| wt ALP | 32 |
| MA192 | 31 |
| MA213 | 52 |
| ML213 | 45 |
| MS213 | 44 |
| MT213 | 46 |
| MG213 | 44 |
| MS192 | 48 |
| MS213/VA217a | 53 |

LSD (least significant difference) = 7.0%

Performance of the alpha-lytic variants, with the exception of MA192, was equivalent to Savinase® against grass stain used at equal weight of protease in the wash liquor.

Example 6. Stability to Oxidation

Differential scanning calorimetry was used to examine the effect of oxidation on the stability of alpha-lytic protease and the mutants MA192 and MA213. The enzymes were incubated with 0.5% hydrogen peroxide in 50 mM acetate, 10 mM $CaCl_2$, pH 5 for 16 hour at 25°C. Unreacted hydrogen peroxide was destroyed by the addition of a small amount of catalase. Denaturation temperatures were determined as in Example 4. The results are shown in Table 8.

## Table 8

| | $T_m$ (°C) | | $\Delta$ $T_m$ |
|---|---|---|---|
| | native | oxidized | (native-oxidized) |
| wt ALP | 78.5 | 71.5 | 7.0 |
| MA192 | 79.1 | 76.3 | 2.8 |
| MA213 | 83.0 | 76.0 | 7.0 |

The decrease in denaturation temperatures for the oxidized enzymes indicates that oxidation causes a decrease in stability for each enzyme. However, due to the increased stability of the unoxidized forms of both mutants MA192 and MA213, the oxidized forms of these enzymes are nearly as stable as the unoxidized wild-type enzyme. Further, the smaller change in $T_m$ upon oxidation for MA192 shows that substitution of methionine with an oxidant-insensitive residue at this position results in increased stability to oxidation.

The stability to oxidation of alpha-lytic protease and the mutants MA192 and MA213 was compared to Alcalase® and Savinase® by examining the effect on the hydrolytic activity towards the preferred synthetic peptide substrate for each enzyme. The activity was assayed as described in the hydrolytic activity assay after incubation of equal amounts of the enzymes at 25°C for 1 hour in 50 mM Bicine, 10 mM $CaCl_2$, pH 8 in the presence of 2% hydrogen peroxide. The results are presented in Table 9.

## Table 9

### % of initial activity

| | |
|---|---|
| wt ALP | 39 |
| MA192 | 89 |
| MA213 | 95 |
| Alcalase® | 52 |
| Savinase® | 12 |

The data in Table 9 shows that wild-type ALP and its variants show increased stability to oxidation compared to Savinase®. In addition, substitution of methionine at either position 192 or 213 with an oxidant-insensitive residue results in increased stability to oxidation. Both alpha-lytic protease variants MA192 and MA213 retain significantly more activity than either Alcalase® or Savinase® in the presence of an oxidant.

Example 7. Effect of Calcium on Solution Stability of ALP

The subtilisin proteases have one or more calcium binding sites. The presence of millimolar levels of $Ca^{+2}$ is necessary for reasonable stability of these proteases, Adebodun and Jordan (1989) Biochem 28:7524-7531. No calcium binding site is apparent in ALP on examination of the crystal structure.

Differential scanning calorimetry was used to demonstrate the lack of dependence of ALP stability on the presence of calcium ($Ca^{+2}$). The dependence of Savinase® stability on $Ca^{+2}$ was examined for comparison. Calorimetric measurements were made as described in Example 4. Protein solutions were prepared in 50 mM acetate, pH 5, and 50 mM Bicine, pH 8, in the presence and absence of 10 mM $CaCl_2$ at both pH values.

The denaturation temperatures for wild-type ALP and Savinase® are listed in Table 10. For Savinase®, at both pH 5 and pH 8 there is a significant increase in the $T_m$ when $Ca^{+2}$ is present. In contrast, there is no difference in the $T_m$ values for ALP in the presence and absence of $Ca^{+2}$, at either pH. The $Ca^{+2}$ independence of ALP provides an advantage over the subtilising in the formulation of cleaning compositions in the presence of builders which sequester $Ca^{+2}$.

### Table 10

| pH | | $T_m(°C)$ wt ALP | $T_m(°C)$ Savinase |
|---|---|---|---|
| 5 | $-Ca^{++}$ | 78.6 | 62.0 |
| | $+Ca^{++}$ | 78.6 | 73.4 |
| 8 | $-Ca^{++}$ | 73.2 | 64.1 |
| | $+Ca^{++}$ | 73.2 | 71.4 |

Example 8. Washing performance of Inventive and Commercial Protease

The stain removal benefit was measured in automatic washing machines using cotton fabric stained with grass extract. These experiments were performed using a standard powdered detergent under the following conditions: Wash temperature of 95°C and for 12 minutes with a rinse temperature of 68°C. Four replicate stains were used per treatment. The results of these experiments, expressed as % stain removal at wash levels typical of manufactured detergents, are shown in Table 11.

## Table 11

| TREATMENT | % STAIN REMOVAL |
|---|---|
| Detergent Only | 39% |
| Savinase 0.5 ug/ml | 90% |
| MA213 0.5 ug/ml | 89% |
| Savinase 0.7 ug/ml | 91% |
| MA213 0.7 ug/ml | 91% |

The result of these studies show that the performance of the inventive protease (MA213) is equivalent to the most widely used commercial product.

## SEQUENCE LISTING

(1)   GENERAL INFORMATION:

    (i)   APPLICANT: Anderson, Susan
                        Ottoboni, Susan
                        Tamm, Joseph

    (ii)   TITLE OF INVENTION: Alpha-Lytic Protease Cleaning Compositions

    (iii)   NUMBER OF SEQUENCES: 2

    (iv)   CORRESPONDENCE ADDRESS:
        (A) ADDRESSEE: McCutchen, Doyle, Brown & Enersen
        (B) STREET: Three Embarcadero Center
        (C) CITY: San Francisco
        (D) STATE: California
        (E) COUNTRY: USA
        (F) ZIP: 94111

```
      (v)   COMPUTER READABLE FORM:
            (A) MEDIUM TYPE: Floppy disk
            (B) COMPUTER: IBM PC compatible
            (C) OPERATING SYSTEM: PC-DOS/MS-DOS
            (D) SOFTWARE: PatentIn Release #1.24

      (vi)  CURRENT APPLICATION DATA:
            (A) APPLICATION NUMBER: US
            (B) FILING DATE:
            (C) CLASSIFICATION:

    (viii)  ATTORNEY/AGENT INFORMATION:
            (A) NAME: Murphy, Lisabeth Feix
            (B) REGISTRATION NUMBER: 31,547
            (C) REFERENCE/DOCKET NUMBER: 17652-002

      (ix)  TELECOMMUNICATION INFORMATION:
            (A) TELEPHONE: (415) 393-2403
            (B) TELEFAX: (415) 393-2288


(2)  INFORMATION FOR SEQ ID NO:1:

      (i)   SEQUENCE CHARACTERISTICS:
            (A) LENGTH: 1194 base pairs
            (B) TYPE: nucleic acid
            (C) STRANDEDNESS: single
            (D) TOPOLOGY: linear

      (ii)  MOLECULE TYPE: DNA (genomic)

     (iii)  HYPOTHETICAL: N

      (iv)  ANTI-SENSE: N

      (ix)  FEATURE:
            (A) NAME/KEY: -
            (B) LOCATION: 1005
            (D) OTHER INFORMATION: /note= "The residue here
                                          may be C or G."
      (ix) FEATURE:
            (A) NAME/KEY: -
            (B) LOCATION: 1009
            (D) OTHER INFORMATION: /note= "The residue here
                                          may be A, T, C or G."
      (ix) FEATURE:
            (A) NAME/KEY: -
            (B) LOCATION: 1010
            (D) OTHER INFORMATION: /note= "The residue here
                                          may be A, C, G or T."
```

```
(ix) FEATURE:
        (A) NAME/KEY: -
        (B) LOCATION: 1011
        (D) OTHER INFORMATION: /note= "The residue here
                                      may be T, C, G or A."
(ix) FEATURE:
        (A) NAME/KEY: -
        (B) LOCATION: 1069
        (D) OTHER INFORMATION: /note= "The residue here
                                      may be A, T, C or G."
(ix) FEATURE:
        (A) NAME/KEY: -
        (B) LOCATION: 1070
        (D) OTHER INFORMATION: /note= "The residue here
                                      may be A, C, G or T."
(ix) FEATURE:
        (A) NAME/KEY: -
        (B) LOCATION: 1071
        (D) OTHER INFORMATION: /note= "The residue here
                                      may be T, C, A or G."
(ix) FEATURE:
        (A) NAME/KEY: -
        (B) LOCATION: 1084
        (D) OTHER INFORMATION: /note= "The residue here
                                      may be A, T, C or G."
(ix) FEATURE:
        (A) NAME/KEY: -
        (B) LOCATION: 1085
        (D) OTHER INFORMATION: /note= "The residue here
                                      may be A, C, G or T."
(ix) FEATURE:
        (A) NAME/KEY: -
        (B) LOCATION: 1086
        (D) OTHER INFORMATION: /note= "The residue here
                                      may be C, G, T or A."
(ix)    FEATURE:
        (A) NAME/KEY: -
        (B) LOCATION: 1129
        (D) OTHER INFORMATION: /note= "The residue here
                                      may be T or A."
(ix)    FEATURE:
        (A) NAME/KEY: -
        (B) LOCATION: 1130
        (D) OTHER INFORMATION: /note= "The residue here
                                      may be C or G."
(ix)    FEATURE:
        (A) NAME/KEY: -
        (B) LOCATION: 1131
        (D) OTHER INFORMATION: /note= "The residue here
                                      may be G or C."
```

```
(ix) FEATURE:
        (A) NAME/KEY: -
        (B) LOCATION: 1133
        (D) OTHER INFORMATION: /note= "The residue here
                                        may be C or G."
(ix) FEATURE:
        (A) NAME/KEY: CDS
        (B) LOCATION: 1..1194
        (D) OTHER INFORMATION:


    (xi) SEQUENCE DESCRIPTION: SEQ ID NO:1:

ATGTACGTAT CGAACCACCG TTCGCGCCGC GTCGCGCGCG TGTCCGTTTC CTGCCTCGTC    60

GCCGCGCTGG CGGCGATGTC CTGCGGCGCT GCGCTGGCGG CCGATCAGGT CGATCCTCAG   120

CTAAAATTCG CAATGCAGCG CGATCTGGGC ATCTTCCCGA CCCAGCTGCC GCAGTACCTG   180

CAGACCGAAA AACTCGCGCG CACCCAGGCT GCGGCGATCG AGCGCGAGTT CGGTGCTCAG   240

TTCGCCGGCA GCTGGATCGA GCGCAACGAA GACGGCAGCT TCAAGCTCGT CGCCGCGACC   300

TCGGGCGCGC GCAAGTCGAG CACGCTGGGC GGGGTGGAAG TGCGCAACGT GCGCTACAGC   360

CTCAAGCAGC TGCAGTCGGC GATGGAGCAG CTCGACGCCG GTGCCAACGC GCGGGTGAAG   420

GGCGTCAGCA AGCCGCTCGA CGGCGTGCAG AGCTGGTACG TGGATCCGCG CAGCAACGCG   480

GTGGTGGTCA AAGTCGACGA CGGCGCGACG GATGCCGGCG TCGACTTCGT CGCCCTCAGC   540

GGCGCCGACA GTGCGCAGGT GCGGATCGAA AGCTCGCCGG GCAAGCTGCA GACCACGGCC   600

AACATCGTCG GCGGCATCGA ATACTCGATC AACAACGCCT CGCTGTGCTC GGTCGGCTTC   660

TCGGTCACCC GCGGCGCGAC CAAGGGCTTC GTCACCGCCG GCCACTGCGG CACGGTCAAT   720

GCGACCGCGC GCATCGGCGG CGCGGTGGTC GGCACCTTCG CCGCGCGCGT GTTCCCCGGC   780

AACGACCGCG CCTGGGTCAG CCTGACCAGC GCGCAGACTC TGCTGCCGCG CGTGGCCAAC   840

GGCAGCAGCT TCGTCACCGT GCGCGGCAGC ACCGAGGCCG CGGTCGGCGC GGCGGTGTGC   900

CGCTCGGGCC GCACCACCGG TTACCAGTGC GGCACCATCA CCGCCAAGAA CGTCACCGCC   960

AACTACGCCG AAGGCGCGGT GCGCGGCCTG ACCCAGGGCA ACGCNTGCNN NGGCCGCGGC  1020

GATTCGGGCG GTTCGTGGAT CACCAGCGCC GGCCAGGCGC AGGGCGTGNN NTCGGGCGGC  1080
```

AACNNNCAGT CCAACGGCAA CAACTGCGGC ATCCCGGCCT CGCAGCGCNN NANCCTGTTC 1140

GAGCGTCTGC AGCCGATCCT GAGCCAGTAC GGCCTGAGCC TGGTCACCGG CTGA      1194

(2)  INFORMATION FOR SEQ ID NO:2:

      (i)    SEQUENCE CHARACTERISTICS:
         (A) LENGTH: 198 amino acids
         (B) TYPE: amino acid
         (D) TOPOLOGY: linear

      (ii)   MOLECULE TYPE: protein

      (ix)   FEATURE:
         (A) NAME/KEY: Modified-site
         (B) LOCATION: 138
         (D) OTHER INFORMATION: /note= "The amino acid
                              here may be Met, Gly, Phe,
                              Ser, Ala or Asp."

      (ix) FEATURE:
         (A) NAME/KEY: Modified-site
         (B) LOCATION: 158
         (D) OTHER INFORMATION: /note= "The amino acid
                              here may be Met, Gly, Ser
                              Ala, Leu, Thr, Val, Phe or
                              Asp."

      (ix)   FEATURE:
         (A) NAME/KEY: Modified-site
         (B) LOCATION: 163
         (D) OTHER INFORMATION: /note= "The amino acid
                              here may be Val, Ser,
                              Ala, Phe or Asp."

(xi) SEQUENCE DESCRIPTION: SEQ ID NO:2:

Ala Asn Ile Val Gly Gly Ile Glu Tyr Ser Ile Asn Asn Ala Ser Leu
1               5                   10                  15

Cys Ser Val Gly Phe Ser Val Thr Arg Gly Ala Thr Lys Gly Phe Val
        20                  25                  30

Thr Ala Gly His Cys Gly Thr Val Asn Ala Thr Ala Arg Ile Gly Gly
        35                  40                  45

Ala Val Val Gly Thr Phe Ala Ala Arg Val Phe Pro Gly Asn Asp Arg
     50                  55                  60

```
Ala Trp Val Ser Leu Thr Ser Ala Gln Thr Leu Leu Pro Arg Val Ala
65                   70            75                      80

Asn Gly Ser Ser Phe Val Thr Val Arg Gly Ser Thr Glu Ala Ala Val
                85              90                  95

Gly Ala Ala Val Cys Arg Ser Gly Arg Thr Thr Gly Tyr Gln Cys Gly
            100             105             110

Thr Ile Thr Ala Lys Asn Val Thr Ala Asn Tyr Ala Glu Gly Ala Val
        115             120             125

Arg Gly Leu Thr Gln Gly Asn Ala Cys Xaa Gly Arg Gly Asp Ser Gly
    130             135             140

Gly Ser Trp Ile Thr Ser Ala Gly Gln Ala Gln Gly Val Xaa Ser Gly
145             150             155             160

Gly Asn Xaa Gln Ser Asn Gly Asn Asn Cys Gly Ile Pro Ala Ser Gln
            165             170             175

Arg Ser Ser Leu Phe Glu Arg Leu Gln Pro Ile Leu Ser Gln Tyr Gly
            180             185             190

Leu Ser Leu Val Thr Gly
        195
```

## Claims

1. A cleaning composition characterised in that it comprises a surfactant and, in an amount effective to enhance removal of protein-containing materials, an alkaline serine protease having the following sequence (SEQ ID No. 2):

$$[AA_{1-137}]\text{-}Xaa_{138}\text{-}[AA_{139-157}]\text{-}Xaa_{158}\text{-}[AA_{159-162}]\text{-}Xaa_{163}\text{-}[AA_{164-198}]$$

wherein

$AA_{1-137}$ represents amino acids 1 to 137 of native alpha-lytic protease;

$Xaa_{138}$ represents methionine, serine or alanine;

$AA_{139-157}$ represents amino acids 139 to 157 of native alpha-lytic protease;

$Xaa_{158}$ represents methionine, alanine, threonine, glycine, serine or leucine;

$AA_{159-162}$ represents amino acids 159 to 162 of native alpha-lytic protease;

$Xaa_{163}$ represents valine or alanine; and

$AA_{164-198}$ represents amino acids 164 to 198 of native alpha-lytic protease.

2. A cleaning composition as claimed in claim 1 wherein the alkaline protease has the following characteristics:
   (a) an optimum hydrolytic activity at a pH of from 7 to 12;
   (b) a stable activity in aqueous solution over a pH range of from 5 to 10.5, preferably from 5 to 8; and
   (c) a melting temperature ($T_m$), greater than 75 °C at pH 5 and preferably greater than 73°C at pH 8.

3. A cleaning composition as claimed in claim 1 or claim 2 wherein the alkaline protease is derived from the genus Lysobacter, preferably from Lysobacter enzymogenes.

4. A cleaning composition as claimed in any of claims 1 to 3 wherein the residue at position 138 is alanine or serine, preferably serine.

5. A cleaning composition as claimed in any of claims 1 to 4 wherein the residue at position 158 is alanine, glycine, threonine, serine or leucine.

6. A cleaning composition as claimed in any of claims 1 to 5 wherein the residue at position 163 is alanine or valine.

7. A cleaning composition as claimed in any of claims 1 to 6 wherein the protease is calcium-independent and/or is stable in the presence of a peroxide.

8. A cleaning composition as claimed in any of claims 1 to 7 which is a laundry detergent or additive composition, which optionally comprises at least one surfactant and which may be a liquid or dry formulation.

9. A cleaning composition as claimed in any of claims 1 to 8 wherein it is an automatic dishwashing composition.

10. A method of cleaning characterised in that it comprises contacting a surface soiled with proteinaceous material with a cleaning effective amount of a cleaning composition as claimed in any of claims 1 to 9, optionally in solution.

11. A method as claimed in claim 10 wherein the surface is a textile material or the surface is selected from dishes, glassware, eating and serving utensils and food containers.

Eag1 4468
NOT1 4467

SPH1 4377
NSP75241 4377

Eag1 4000
NRU1 3967

Nar1 3734
BSTX1 3664

Eag1 155

Bamh1 519
Eag1 155

Pvu1 272

Bamh1 519
Nar1 598

Sac2 726

BAL1 890
Sac2 935

MLU1 105:
Sac2 1071

XHO1 1185

XBal/BamH1 12

NCO1 1322

ASU2 1550

ECO0109 1684

AATII 1742

PhoA

pro-lytic

mat-lytic

palp 5
4581 bp

rep fxns

HGIE2 2966

amp

Pvu1 2293

XMN1 2061

| | PhoA: | 1 | 150 |
|---|---|---|---|
| | pro-lytic: | 155 | 653 |
| | mat-lytic: | 654 | 1273 |
| | amp: | 1947 | 2734 |
| | rep fxns: | 2974 | 3274 |

FIG. 1

EP 0 506 448 A1

```
                                      27                                              54
ATG TAC GTA TCG AAC CAC CGT TCG CGC CGC GTC GCG CGC GTG TCC GTT TCC TGC
 M   Y   V   S   N   H   R   S   R   R   V   A   R   V   S   V   S   C
(-198)
                                      81                                             108
CTC GTC GCC GCG CTG GCG GCG ATG TCC TGC GGC GCT GCG CTG GCG GCC GAT CAG
 L   V   A   A   L   A   A   M   S   C   G   A   A   L   A   A   D   Q

                                     135                                             162
GTC GAT CCT CAG CTA AAA TTC GCA ATG CAG CGC GAT CTG GGC ATC TTC CCG ACC
 V   D   P   Q   L   K   F   A   M   Q   R   D   L   G   I   F   P   T

                                     189                                             216
CAG CTG CCG CAG TAC CTG CAG ACC GAA AAA CTC GCG CGC ACC CAG GCT GCG GCG
 Q   L   P   Q   Y   L   Q   T   E   K   L   A   R   T   Q   A   A   A

                                     243                                             270
ATC GAG CGC GAG TTC GGT GCT CAG TTC GCC GGC AGC TGG ATC GAG CGC AAC GAA
 I   E   R   E   F   G   A   Q   F   A   G   S   W   I   E   R   N   E

                                     297                                             324
GAC GGC AGC TTC AAG CTC GTC GCC GCG ACC TCG GGC GCG CGC AAG TCG AGC ACG
 D   G   S   F   K   L   V   A   A   T   S   G   A   R   K   S   S   T

                                     351                                             378
CTG GGC GGG GTG GAA GTG CGC AAC GTG CGC TAC AGC CTC AAG CAG CTG CAG TCG
 L   G   G   V   E   V   R   N   V   R   Y   S   L   K   Q   L   Q   S

                                     405                                             432
GCG ATG GAG CAG CTC GAC GCC GGT GCC AAC GCG CGG GTG AAG GGC GTC AGC AAG
 A   M   E   Q   L   D   A   G   A   N   A   R   V   K   G   V   S   K
```

**FIG. 2A**

```
                                        459                                        486
CCG CTC GAC GGC GTG CAG AGC TGG TAC GTG GAT CCG CGC AGC AAC GCG GTG GTG
 P   L   D   G   V   Q   S   W   Y   V   D   P   R   S   N   A   V   V

                                        513                                        540
GTC AAA GTC GAC GAC GGC GCG ACG GAT GCC GGC GTC GAC TTC GTC GCC CTC AGC
 V   K   V   D   D   G   A   T   D   A   G   V   D   F   V   A   L   S

                                        567                                        594
GGC GCC GAC AGT GCG CAG GTG CGG ATC GAA AGC TCG CCG GGC AAG CTG CAG ACC
 G   A   D   S   A   Q   V   R   I   E   S   S   P   G   K   L   Q   T

                                        621                                        648
ACG GCC AAC ATC GTC GGC GGC ATC GAA TAC TCG ATC AAC AAC GCC TCG CTG TGC
 T   A   N   I   V   G   G   I   E   Y   S   I   N   N   A   S   L   C
    (+1)
                                        675                                        702
TCG GTC GGC TTC TCG GTC ACC CGC GGC GCG ACC AAG GGC TTC GTC ACC GCC GGC
 S   V   G   F   S   V   T   R   G   A   T   K   G   F   V   T   A   G

                                        729                                        756
CAC TGC GGC ACG GTC AAT GCG ACC GCG CGC ATC GGC GGC GCG GTG GTC GGC ACC
 H   C   G   T   V   N   A   T   A   R   I   G   G   A   V   V   G   T

                                        783                                        810
TTC GCC GCG CGC GTG TTC CCC GGC AAC GAC CGC GCC TGG GTC AGC CTG ACC AGC
 F   A   A   R   V   F   P   G   N   D   R   A   W   V   S   L   T   S

                                        387                                        864
GCG CAG ACT CTG CTG CCG CGC GTG GCC AAC GGC AGC AGC TTC GTC ACC GTG CGC
 A   Q   T   L   L   P   R   V   A   N   G   S   S   F   V   T   V   R
```

**FIG. 2B**

```
                              891                                                   918
GGC AGC ACC GAG GCC GCG GTC GGC GCG GCG GTG TGC CGC TCG GGC CGC ACC ACC
 G   S   T   E   A   A   V   G   A   A   V   C   R   S   G   R   T   T

                              945                                                   972
GGT TAC CAG TGC GGC ACC ATC ACC GCC AAG AAC GTC ACC GCC AAC TAC GCC GAA
 G   Y   Q   C   G   T   I   T   A   K   N   V   T   A   N   Y   A   E

                                               MluI
                              999          ⌐‾‾‾‾‾⌐        ⌐‾‾‾‾⌐                     1026
GGC GCG GTG CGC GGC CTG ACC CAG GGC AAC GCC TGC |ATG| GGC CGC GGC GAT TCG
 G   A   V   R   G   L   T   Q   G   N   A   C  | M |  G   R   G   D   S

                              1053                          ⌐‾‾‾‾⌐                  1080
GGC GGT TCG TGG ATC ACC AGC GCC GGC CAG GCG CAG GGC GTG |ATG| TCG GGC GGC
 G   G   S   W   I   T   S   A   G   Q   A   Q   G   V  | M |  S   G   G

                                                                 XhoI 1134
                              1107                              ⌐‾‾‾‾‾⌐
AAC |GTG| CAG TCC AAC GGC AAC AAC TGC GGC ATC CCG GCC TCG CAG CGC AGC AGC
 N  | V |  Q   S   N   G   N   N   C   G   I   P   A   S   Q   R   S   S

                              1161                                                  1188
CTG TTC GAG CGT CTG CAG CCG ATC CTG AGC CAG TAC GGC CTG AGC CTG GTC ACC
 L   F   E   R   L   Q   P   I   L   S   Q   Y   G   L   S   L   V   T

GGC TGA
 G
```

**FIG. 2C**

FIG. 3

# EUROPEAN SEARCH REPORT

| | European Patent Office | | Application Number |
|---|---|---|---|

EP     92 30 2700

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | US-A-4 421 664 (C.R. ANDERSON ET AL.) <br> * column 5, line 48 – column 6, line 24; example 6 * <br> --- | 1,2,7-11 | C11D3/386 <br> C12N9/52 <br> C12N9/56 |
| A | JOURN. MOL. BIOL. <br> no. 183, 1985, <br> pages 479 – 502; <br> M. FUJINAGA ET AL.: 'Refined Structure of §-Lytic Protease at 1.7 A Resolution' <br> * the whole document * <br> --- | 3 | |
| D,A | NATURE <br> vol. 339, 18 May 1989, <br> pages 191 – 195; <br> R. BONE ET AL.: 'Structural plasticy broadens the specifity of an engineered protease' <br> * the whole document * <br> --- | 3 | |
| A | CHEMICAL ABSTRACTS, vol. 105, no. 9, <br> November 1986, Columbus, Ohio, US; <br> abstract no. 155085, <br> * abstract * <br> & FRAGRANCE J. <br> vol. 13, 1985, <br> pages 79 – 81; <br> O. TAKASHI: 'A new protease for low temperature washing' <br> ----- | 1,2,8 | **TECHNICAL FIELDS SEARCHED (Int. Cl.5 )** <br><br> C11D <br> C12N |

The present search report has been drawn up for all claims

| Place of search | Date of completion of the search | Examiner |
|---|---|---|
| THE HAGUE | 08 JULY 1992 | GRITTERN A.G. |